# EUROPEAN PATENT SPECIFICATION

(11) **EP 1 874 288 B1**
(45) Date of publication and mention of the grant of the patent: **17.07.2013**
(21) Application number: 06754750.5
(22) Date of filing: 18.04.2006
(51) Int. Cl.: A61K 31/221, A61P 25/02

(54) **ACETYL L-CARNITINE FOR PREVENTING PAINFUL PERIPHERAL DIABETIC NEUROPATHY**
ACETYL L-CARNITIN ZUR VORBEUGUNG SCHMERZHAFTER PERIPHÄRER DIABETISCHER NEUROPATHIE
ACÉTYL-L-CARNITINE POUR LA PRÉVENTION DE LA NEUROPATHIE PÉRIPHÉRIQUE DIABÉTIQUE DOULOUREUSE

(30) Priority: 26.04.2005 EP 05009056; 26.04.2005 US 114224
(43) Date of publication of application: 09.01.2008
(73) Proprietor: SIGMA-TAU Industrie Farmaceutiche Riunite S.p.A., 00144 Roma (IT)
(72) Inventor: CALVANI, Menotti, I-00146 Rome (IT); AMATO, Antonino, North Bethesda, Maryland 20852 (US)
(74) Representative: Spadaro, Marco
(86) International application number: PCT/EP2006/061632
(87) International publication number: WO 2006/114372

(56) References cited:
- EP-A- 0 256 999
- GRANDIS DE D ET AL: "L-ACETYLCARNITINE IN THE TREATMENT OF PATIENTS WITH PERIPHERAL NEUROPATHIES A SHORT TERM, DOUBLE-BLIND CLINICAL STUDY OF 426 PATIENTS" CLINICAL DRUG INVESTIGATION, ADIS INTERNATIONAL, AUCKLAND, NZ, vol. 10, no. 6, 1995, pages 317-322, XP001106363 ISSN: 1173-2563 cited in the application
- QUATRARO A ET AL: "ACETYL-L-CARNITINE FOR SYMPTOMATIC DIABETIC NEUROPATHY" DIABETOLOGIA, BERLIN, DE, vol. 38, no. 1, 1995, page 123, XP009050921 ISSN: 0012-186X cited in the application
- GIULIO DI A M ET AL: "ACETYL-L-CARNITINE PREVENTS SUBSTANCE P LOSS IN THE SCIATIC NERVE AND LUMBAR SPINAL CORD OF DIABETIC ANIMALS", PROCEEDINGS MEMBRANE TECHNOLOGY SEPARATIONS PLANNING CONF, XX, XX, vol. 12, no. 5/06, 1 January 1992 (1992-01-01), pages 243-246, XP001106182,

## Description

The present invention relates to the use of acetyl L-carnitine (ALC) for the preparation of a medicament for the prevention of painful peripheral neuropathy, characterised by pain, paraesthesia or hyperaesthesia, in patients suffering from type 2 diabetes.

Diabetic neuropathy is the most frequent peripheral neuropathy in the western world and includes different forms of neuropathy, the most common of which is diabetic polyneurbpathy.

The anatomico-pathological picture of diabetic peripheral neuropathy consists in a focal or widespread non-specific loss of fibres, with demyelination associated with structural or endoneuronal abnormalities of the connective tissue or small vessels.

Various metabolic abnormalities and biochemical changes have been documented both in experimental models of diabetes and in diabetic patients, including an increase in glucose metabolism and a reduction in myoinositol.

The characteristic symptoms of diabetic polyneuropathy consist in the presence burning or lancinating pain accompanied by clinical signs of symmetrical impairment of sensitivity, motility, and/or deep tendon reflexes, such symptoms being predominant in the distal segments of the lower limbs.

Diabetic peripheral neuropathy is caused by hyperglycaemia, and metabolic imbalance secondary to hyperglycaemia and ischaemia of the vasa nervorum are the best known pathogenetic mechanisms.

Many variables may speed up or reduce the times when the characteristic symptoms of diabetic peripheral neuropathy in general, and painful peripheral neuropathy in particular, set in; for example, good metabolic control of blood serum glucose levels can certainly delay the onset of said symptoms.

The use of acetyl L-carnitine for the prevention of painful peripheral, neuropathy in patients suffering from type 2 diabetes has never been previously described.

In the literature one can find numerous publications that seek to demonstrate or clarify the therapeutic role of acetyl L-carnitine in the treatment of a number of symptoms, including neuropathic pain, in the course of diabetic neuropathy, but these publications do not claim or suggest that acetyl L-carnitine is a useful compound for the prevention of painful peripheral neuropathy in patients suffering from type 2 diabetes.

In Diabetes Care, 2005; Jan; 28(1): 89-94 it is reported that treatment with ALC is useful for relieving the pain symptoms and for regenerating nerve fibres, in patients suffering from stabilised diabetic neuropathy.

In "Giornale Italiano di Diabetologia, 1998, V.18, 30-31" the use of acetyl L-carnitine is described for the treatment of neuropathic pain in patients suffering from sensorimotor polyneuropathy.

In Drugs in Research and Development 2002, Vol 3 (4), pp 223-31, the use of acetyl L-carnitine is described for the treatment of neuropathic pain in patients with diabetic neuropathy.

In Diabetologia 1995, VOL/ISS/PG. 38/1 (123) the use of acetyl L-carnitine is described for the treatment of neuropathic pain in patients suffering from diabetic neuropathy.

In "IL GIORNALE DEI CONGRESSI MEDICI, 5, 14-19,1993" the use of acetyl L-carnitine is described for the treatment of neuropathic pain in patients suffering from diabetic neuropathy on treatment with insulin or oral antidiabetic agents.

In J. of the American Diabetes Association June 2002, Vol 51, Supplement 2, the use of acetyl L-carnitine is described for the treatment of neuropathic pain in patients suffering from diabetic neuropathy.

In CONGRESSO NAZIONALE DELLA SOCIETA ITALIANA DI NEUROFISIOLOGIA CLINICA, ABSTR ACTS, PERUGIA 1-4 June, 1994, p 98, the use of acetyl L-carnitine is described for the treatment of neuropathic pain in patients suffering from diabetic neuropathy.

In Clin. Drug. Invest, Vol 10 (6), pp 317-22 1995, the use of acetyl L-carnitine is described for the treatment of neuropathic pain in patients suffering from diabetic neuropathy.

In Int. J. Clin. Pharm. Res. (XII), 5/6; 243, acetyl-L-carnithine prevents substance P loss in the sciatic nerve and lumbor spinal cord of diabetic animals,

In Int. J. Clin. Pharm. Res. XV (1):9-15; 995, the use of acetyl L-carnitine is described for the treatment of pain in patients suffering from diabetic neuropathy.

In Journal of the Neurological Sciences, 1997, Suppl. to Vol 150 it is reported that acetyl L-carnitine improves nerve conduction velocity in diabetic patients suffering from polyneuropathy.

In IX CONGRESSO NAZIONALE SOCIETA' ITALIANA DI FARMACOLOGIA CLINICA; II CONGRESS MEDITERRANEAN SOCIETY CLINICAL PHARMACOLOGY "THERAPEUTIC ADVANCES AND NEW HEALTH PROBLEMS", VENICE, 8-10/ 10/ 1991 ABS, the effect of acetyl L-carnitine treatment on 500 patients suffering from peripheral neuropathy of various different origins is described.

In Drugs 1997 Sept: 54 (3) 414-421 it is reported that acetyl L-carnitine improves nerve conduction velocity in diabetic patients suffering from neuropathy.

US 4,751,242 describes the use of acetyl L-carnitine for the treatment of neuropathic pain in patients with peripheral neuropathy of various origins, including diabetic peripheral neuropathy.

WO 02096409 refers to the use of acetyl L-carnitine for the preparation of a medicament with "pre-emptive-type" analgesic activity. What is meant by "pre-emptive" analgesia is a therapeutic strategy that involves the early administration of a substance, in relation to the painful event, capable of blocking the entry of the pain stimulus into the central nervous system, thus preventing the facilitating response evoked by the nociceptive impulse to the spinal cord. The efficacy of a "pre-emptive" analgesic drug according to the description in WO 02096409 depends not only on the time treatment is initiated in relation to the painful event but also on the effective ability of the drug to prevent alterations of central pain sensitisation mechanisms.

WO 02096409, as an example of preventive pain therapy, indicates the prevention of pain that occurs after a surgical operation.

In these studies, there is never any description or suggestion of the use of ALC for the prevention of painful peripheral neuropathy in patients suffering from type 2 diabetes.

In the medical field no drugs are known which are useful for the prevention of painful peripheral neuropathy in patients suffering from type 2 diabetes.

It has now been found that acetyl L-carnitine lends itself to being employed as a useful agent for the preparation of a medicament for the prevention of painful peripheral neuropathy in patients suffering from type 2 diabetes.

The object of the present invention is therefore the use of acetyl L-carnitine, or one of its pharmaceutically acceptable salts, for the preparation of a medicament for the prevention of painful peripheral neuropathy in patients suffering from type 2 diabetes, in which said painful peripheral neuropathy is characterised by symptoms selected from the group consisting of pain, paraesthesia and hyperaesthesia.

What is meant by pharmaceutically acceptable salt of acetyl L-carnitine is any salt of the latter with an acid that does not give rise to unwanted toxic effects. These acids are well known to pharmacologists and to experts in pharmaceutical technology.

Examples of such salts are, for example, chloride, bromide, orotate, acid aspartate, acid citrate, magnesium citrate, acid phsophate, fumarate and acid fumarate, magnesium fumarate, lactate, maleate and acid maleate, mucate, acid oxalate, pamoate, acid pamoate, acid sulphate, glucose phosphate, tartrate, acid tartrate, magnesium tartrate, 2-amino ethanesulphonate, magnesium 2-amino ethanesulphonate, choline tartrate and trichloroacetate.

The following examples illustrate the invention.

### EXAMPLE 1 (Clinical Trial BM 14329)

A multicentre, randomised, double-blind, placebo-controlled clinical trial of 52 weeks' duration was conducted in patients with type 2 diabetes suffering from diabetic peripheral neuropathy.

The patients were treated with acetyl L-carnitine at a dose of 0.5 grams or 1 gram, three times daily, or with placebo for 52 weeks.

The patients recruited into the trial were patients of male and female sex and were aged from 18 to 70 years, in whom diabetes had been diagnosed more than one year earlier and presenting HbA1c greater than 5.9% .

After 12, 26 and 52 weeks of treatment, the presence and intensity of pain, paraesthesia and hyperaesthesia, amongst other variables, were assessed as characteristic symptoms of painful peripheral neuropathy in patients who at the time of recruitment presented no such symptoms.

At the end of the clinical trial, statistical analysis of the results obtained showed that treatment with ALC for 26 or 52 weeks was capable of curing the symptoms of diabetic peripheral neuropathy that were already present at the start of the clinical trial. The curative effect was not the same in all the population treated, but was better only in patients with type 2 diabetes.

Further processing of the experimental data in this clinical trial showed, unexpectedly and surprisingly, that acetyl L-carnitine was capable not only of curing but also of preventing the symptoms typical of diabetic painful peripheral neuropathy, including pain, hyperaesthesia and paraesthesia, in patients who at entry into the trial did not present such symptoms.

Even more surprising was the discovery that said preventive activity was present only in patients suffering from type 2 diabetes and at the higher dose of the drug administered.

The results in patients with type 2 diabetes treated with ALC at the dose of 3 grams/day are reported in Tables 1-3 here below..

**TABLE 1**

| **Symptom** | **Placebo (n = 152 patients)** | | **Acetyl L-carnitine 3.0 g/day (n = 217 patients)** | | **Significance (*P* value, Fisher Exact Test)** |
|---|---|---|---|---|---|
| | Basa 1 | 12 weeks | Basal | 12 weeks | PLACEBO VS ALC |
| Pain | 0% | 27.0% | 0% | 17.1% | 0.027 |
| Pain, hyperaesthesia, paraesthesia | 0% | 50.0% | 0% | 35.5% | 0.007 |

**TABLE 2**

| **Symptom** | **Placebo (n = 152 patients)** | | **Acetyl L-carnitine 3.0 g/day (n = 217 patients)** | | **Significance (*P* value, Fisher Exact Test)** |
|---|---|---|---|---|---|
| | Basal | 26 weeks | Basal | 26 weeks | PLACEBO VS ALC |
| Pain | 0% | 27.0% | 0% | 20.3% | 0.11 |
| Pain, hyperaesthesia, paraesthesia | 0% | 51.6% | 0% | 38,6% | 0.007 |

**TABLE 3**

| **Symptom** | **Placebo (n = 152 patients)** | | **Acetyl L-carnitine 3.0 g/day (n = 217 patients)** | | **Significance (*P* value, Fisher Exact Test)** |
|---|---|---|---|---|---|
| | Basa 1 | 52 weeks | Basal | 52 weeks | PLACEBO VS ALC |
| Pain | 0% | 32.9% | 0% | 29.5% | 0.494 |
| Pain, hyperaesthesia paraesthesia | 0% | 59.2% | 0% | 47.5% | 0.027 |

### EXAMPLE 2 (clinical Trial BM 14330)

A multicentre randomised, double-blind, placebo-controlled clinical trial was conducted with the same characteristics as the clinical trial described in Example 1.

The results obtained in patients with type 2 diabetes treated with ALC at the dose of 3 grams/day, are reported in Tables 4-6 here below.

**TABLE 4**

| **Symptom** | **Placebo (n = 142 patients)** | | **Acetyl L-carnitine 3.0 g/day (n = 181 patients)** | | **Significance (*P* value, Fisher Exact Test)** |
|---|---|---|---|---|---|
| | Basa 1 | 12 weeks | Basal | 12 weeks | PLACEBO VS ALC |
| Pain | 0% | 45.1% | 0% | 27.6% | 0.001 |
| Pain, hyperaesthesia, paraesthesia | 0% | 64.1% | 0% | 42.5% | 0.001 |

**TABLE 5**

| **Symptom** | **Placebo (n = 142 patients)** | | **Acetyl L-carnitine 3.0 g/day (n = 181 patients)** | | **Significance (Fisher Exact Test)** |
|---|---|---|---|---|---|
| | Basa 1 | 12 weeks | Basal | 12 weeks | PLACEBO VS ALC |
| Pain | 0% | 45.8% | 0% | 28.7% | 0.002 |
| Pain, hyperaesthesia, paraesthesia | 0% | 64.8% | 0% | 43.1% | 0.001 |

**TABLE 6**

| **Symptom** | **Placebo (n = 142 patients)** | | **Acetyl L-carnitine 3.0 g/day (n = 181 patients)** | | **Significance (*P* value, Fisher Exact Test)** |
|---|---|---|---|---|---|
| | Basa 1 | 52 weeks | Basal | 52 weeks | PLACEBO VS ALC |
| Pain | 0% | 54.9% | 0% | 35.9% | 0.001 |
| Pain, hyperaesthesia, paraesthesia | 0% | 66.9% | 0% | 48.1% | 0.001 |

On analysing the results reported in Tables 1-6, it can be noted that the compound according to the invention showed a statistically significant ability to prevent the onset of symptoms associated with diabetic painful peripheral neuropathy, such as pain, paraesthesia and hyperaesthesia.

Acetyl L-carnitine is a known compound, whose preparation process is described in US patents 4439438 and 4254053.

The acetyl L-carnitine can be in any form suitable for oral or parenteral administration in human subjects.

On the basis of various factors such as the concentration of active ingredient and the patient's condition, the compound according to the invention can be marketed as a health food supplement, nutritional supplement, or as a therapeutic product on sale subject to obtaining a doctor's prescription or without a doctor's prescription.

It has been found that, though the daily dose of the above-mentioned active ingredient to be administered depends on the patient's age, weight and general condition, on the basis of professional experience, for the prevention of diabetic painful peripheral neuropathy in patients with type 2 diabetes, it is necessary to administer, in multiple doses, an amount of ALC corresponding to at least 3 grams a day, or an equimolar amount of one of its pharmaceutically acceptable salts.

The medicament according to the present invention can be prepared by mixing the active ingredient (acetyl L-carnitine inner salt or one of its pharmaceutically acceptable salts) with excipients suitable for the formulation of compositions for enteral (particularly oral) or parenteral (particularly intramuscular or intravenous) administration.

Said excipients are well known to experts in pharmaceutical technology.

The pharmaceutically acceptable salts of the above-mentioned active ingredients include all pharmaceutically acceptable salts that are prepared by addition of an acid to acetyl L-carnitine inner salt, and that do not give rise to unwanted toxic or side effects. The formation of salts by addition of acids is a well known practice in pharmaceutical technology.

## Claims

1. Use of acetyl L-carnitine, or of one of its pharmaceutically acceptable salts, for the preparation of a medicament for the prevention of painful peripheral neuropathy in patients suffering from type 2 diabetes, in which said painful peripheral neuropathy is **characterised by** symptoms selected from the group consisting of pain, paraesthesia and/ or hyperaesthesia.

2. Use according to claim 1, in which the pharmaceutically acceptable salt of acetyl L-carnitine is selected from the group consisting of chloride, bromide, orotate, acid aspartate, acid citrate, magnesium citrate, acid phsophate, fumarate and acid fumarate, magnesium fumarate, lactate, maleate and acid maleate, mucate, acid oxalate, pamoate, acid pamoate, acid sulphate, glucose phosphate, tartrate, acid tartrate, magnesium tartrate, 2-amino ethanesulphonate, magnesium 2-amino ethanesulphonate, choline tartrate and trichloroacetate.

3. Use according to claim 1, in which the acetyl L-carnitine is administered orally at a dose of at least 3 grams/day.

## Patentansprüche

1. Verwendung von Acetyl-L-carnitin oder eines seiner pharmazeutisch verträglichen Salze zur Herstellung eines Medikaments zur Verhinderung von schmerzhafter peripherer Neuropathie bei Patienten, die unter Typ-2-Diabetes leiden, wobei die genannte schmerzhafte periphere Neuropathie durch Symptome gekennzeichnet ist, die ausgewählt sind aus der Gruppe, bestehend aus Schmerz, Parästhesie und/oder Hyperparästhesie.

2. Verwendung, gemäß Anspruch 1, bei welcher das pharmazeutisch verträgliche Salz von Acetyl-L-carnitin ausgewählt ist aus der Gruppe, bestehend aus Chlorid, Bromid, Orotat, saurem Aspartat, saurem Citrat, Magnesiumcitrat, saurem Phosphat, Fumarat und saurem Fumarat, Magnesiumfumarat, Laktat, Maleat und saurem Maleat, Mucat, saurem Oxalat, Pamoat, saurem Pamoat, saurem Sulfat, Glucosephosphat, Tartrat, saurem Tartrat, Magnesiumtartrat, 2-Aminoethansulfonat, Magnesium-2-aminoethansulfonat, Cholintartrat und Trichloracetat.

3. Verwendung, gemäß Anspruch 1, bei welcher das Acetyl-L-carnitin oral in einer Dosis von wenigstens 3 Gramm/Tag verabreicht wird.

## Revendications

1. Utilisation d'acétyl-L-carnitine, ou d'un de ses sels pharmaceutiquement acceptables, pour la préparation d'un médicament pour la prévention d'une neuropathie périphérique douloureuse chez des patients souffrant de diabète de type 2, ladite neuropathie périphérique douloureuse étant **caractérisée par** des symptômes choisis dans le groupe constitué de douleur, paresthésie et/ou hyperesthésie.

2. Utilisation selon la revendication 1, dans laquelle le sel pharmaceutiquement acceptable d'acétyl-L-carnitine est choisi dans le groupe constitué de chlorure, de bromure, d'orotate, d'aspartate d'acide, de citrate d'acide, de citrate de magnésium, de phosphate d'acide, de fumarate et de fumarate d'acide, de fumarate de magnésium, de lactate, de maléate et de maléate d'acide, de mucate, d'oxalate d'acide, de pamoate, de pamoate d'acide, de sulfate d'acide, de phosphate de glucose, de tartrate, de tartrate d'acide, de tartrate de magnésium, de 2-amino-éthanesulfonate, de 2-amino-éthanesulfonate de magnésium, de tartrate de choline et de trichloroacétate.

3. Utilisation selon la revendication 1, dans laquelle l'acétyl-L-carnitine est administrée par voie orale selon une posologie d'au moins 3 grammes/jour.
